# EUROPÄISCHE PATENTSCHRIFT

(11) **EP 1 981 469 B1**
(45) Veröffentlichungstag und Bekanntmachung des Hinweises auf die Patenterteilung: **03.11.2010**
(21) Anmeldenummer: 07704280.2
(22) Anmeldetag: 31.01.2007
(51) Int. Cl.: A61K 8/02, A61K 8/73, A61K 8/81, A61K 8/86, A61Q 5/06

(54) **KOSMETISCHE HAARGELE MIT HOHER FADENABRISSZEIT**
COSMETIC HAIR GELS WITH HIGH FILAMENT BREAK-UP TIME
GELS COSMETIQUES POUR LES CHEVEUX AYANT UN TEMPS DE RUPTURE DE FILAMENT ELEVE

(30) Priorität: 01.02.2006 DE 102006005451
(43) Veröffentlichungstag der Anmeldung: 22.10.2008
(73) Patentinhaber: Beiersdorf AG, 20253 Hamburg (DE)
(72) Erfinder: DETERT, Marion, 22455 Hamburg (DE); HETZEL, Frank, 21261 Welle (DE); PILZNER, Anke, 22459 Hamburg (DE)
(86) Internationale Anmeldenummer: PCT/EP2007/050958
(87) Internationale Veröffentlichungsnummer: WO 2007/088179

(56) Entgegenhaltungen:
- EP-A- 1 097 701
- WO-A-99/66888
- DE-C1- 19 740 651
- US-A- 5 573 768
- US-B1- 6 177 390

## Beschreibung

Die vorliegende Erfindung betrifft eine gelförmige Zubereitung zur Frisurgestaltung mit besonders hoher Fadenabrisszeit.

Zur temporären Verformung und Stabilisierung der Frisur werden Produkte eingesetzt, die als Stylingmittel bekannt sind. Hierzu zählen u.a. auch Gele.

Üblicherweise bestehen diese Mittel zur Festigung der Haare aus wässrigen oder wässrig alkoholischen Lösungen von verdickenden und filmbildenden natürlichen oder synthetischen Polymeren. Diese Polymere können aus der Gruppe der nichtionischen, kationischen, amphoteren oder anionischen Polymere ausgewählt werden.

Haargele haben insbesondere den für den Verbraucher unangenehmen Nachteil, dass Produkte mit einem sehr hohen Halt, d.h. einem hohen Anteil an festigenden Polymeren in der Formel, auch immer zu Rückständen beim Berühren oder Kämmen der Haare führen. Oft werden diese Rückstände mit Kopfhautschuppen verwechselt. Das entstehen der Rückstände ist darauf zurückzuführen, dass eine hohe Haltestufe auch den Einsatz von einer hohen Konzentration an verdickenden bzw. festigenden Polymeren erfordert.

Die Schrift DE 19907714 offenbart eine Zubereitung zur Behandlung keratinischer Fasern, insbesondere menschlicher Haare, enthaltend eine Wirkstoffkombination aus einem verdickend wirkenden synthetischen kationischen Acryl/Methacrylat-Homopolymeren oder deren Copolymeren und einem weiteren kationischen Polymer, wobei die Zubereitung zwingend ein Tensid enthält, wenn das erste Polymer ein Copolymer ist.

Die Schrift DE 10330609 (BDF) offenbart eine kosmetische Zubereitung in Form einer O/W-Emulsion, die ein mehrfaches Gestalten einer Frisur erlaubt enthaltend mindestens ein festigendes Polymer aus der Gruppe der nichtionischen, anionischen, amphoteren oder kationischen Polymere, zwei nichtionische oberflächenaktive Substanzen (A) und (B), deren HLB-Werte im Fall von (A) zwischen 12 und 15 und im Fall von (B) zwischen 3 und 6 liegen.

Die Schrift WO 2004028496 A1 (Unilever) offenbart wässrige leave-on conditioning Zubereitungen, die 0.01 bis 10% quaternäre Ammoniumverbindungen, 0 bis 10% Siliconöl, 0.00001 bis 0.05% Polyoxyethylen oder polyoxypropylen und 0.01 bis 10% nichtionisches Tensid enthalten und die Zubereitung im Wesentlichen frei von Haarfixiermitteln und kationischen Zellulosederivaten mit vierfachsubstituiertem Stickstoff ist.

Es hat sich für den Fachmann nicht vorhersehbar herausgestellt, daß eine gelförmige kosmetische Zubereitung zur Frisurgestaltung enthaltend ein anionisches verdickendes Polymer, ein oder mehrere filmbildendes Polymer, bevorzugt ein VP/VA-Copolymer, als konditionierendes Polymer, eine kationische Zellulose, ein hochmolekulares Polyethylenglycol mit einer Molmasse von 3000000 bis 5000000 g/mol, bevorzugt 3500000 bis 4500000 g/mol, Wasser mit einer Fadenabrisszeit der Zubereitung gemessen mit einem Capillary Breakup Extensional Rheometer (CaBER™, Thermo Haake) zwischen 1 s und 5,0 s, bevorzugt zwischen 1,5 s und 4,0 , besonders bevorzugt zwischen 2,0 und 3,5, den Mängeln des Standes der Technik abhilft. Durch die Kombination von speziellen verdickenden Polymeren mit einem hochmolekularen PEG wird eine hervorragende, verbesserte Festigungsleistung erzielt die das Haar sehr schnell fixiert, ohne dass Rückstände in erhöhtem Maße auftreten, wie es bei Zubereitungen das Standes der Technik recht oft auftritt und daher zu erwarten wäre. Die so erhaltenen Gele weisen ein ganz spezielles rheologisches Profil auf, welchhes für den Anwender erlebbar ist. Eine solche Zubereitung stellt ein hochviskoses Gel dar, dass sich sehr gut im Haar verteilen lässt und trotz einer hohen Fixierleistung nicht den Nachteil der übermäßig auftretenden Rückstände aufweist. Die Textur des Gels ist sehr speziell und kann je nach Herstellverfahren in der Produktion (Homogenisierung) zu einem Gel mit mehr oder weniger ausgeprägten "Fäden" führen. Die kosmetischen Formulierungen sind weitestgehend frei von Ölen und Tensiden. Das Profil unterscheidet sich in den beschrieben Parametern von herkömmlichen Styling Gelen die derzeit im Markt zu finden sind.

Es wurde weiter gefunden, dass es bevorzugt ist, wenn zusätzlich Alkohol enthalten ist. Besonders bevorzugt ist es, wenn der Gehalt an verdickendem Polymer 0,1 bis 5 Gew.% bezogen auf den Aktivgehalt an Polymer beträgt. Weiter besonders bevorzugt ist es, wenn der Gehalt an filmbildendem Polymer 0,1 bis 5 Gew.% bezogen auf den Aktivgehalt an Polymer beträgt. Weiter besonders bevorzugt ist es, wenn der Gehalt an konditionierndem Polymer 0,1 bis 3 Gew.% bezogen auf den Aktivgehalt an Polymer beträgt. Weiter besonders bevorzugt ist es, wenn der Gehalt an hochmolekularem Polyethylenglycol 0,1 bis 2 Gew.% beträgt. Weiter besonders bevorzugt ist es, wenn der Gehalt an Öl abzüglich Parfümölen 0 bis 0,2 Gew.% beträgt. Weiter besonders bevorzugt ist es, wenn der Gehalt an Tensiden 0,1 bis 2 Gew.% beträgt, wenn in der Formulierung Parfümöl enthalten ist, ansonsten ist nur ein Gehalt von 0 bis 0,5 Gew.% nötig. Weiter besonders bevorzugt ist es, wenn die Zubereitung Carbomer, Polyquaternium-4, VP/VA Copolymer und PEG-90 M enthält. Weiter besonders bevorzugt ist es, wenn die Viskosität der Zubereitung im Bereich 100 bis 300 dPas, bevorzugt 150 bis 250 dPas (gemessen mit Haake VT-02 Viskotester) liegt.

Erfindungsgemäße Zubereitungen können auch bevorzugt keine Emulsionen darstellen, also z.B. besonders bevorzugt einphasig sein.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

### Dehnrheometrie

Die Kenntnis der Dehneigenschaften von Flüssigkeiten ist wichtig, sowohl in der Industrie als auch in der Forschung. Komplexe Strömungen, die starke Dehnkomponenten beinhalten, treten in vielen industriellen Prozessen und Anwendungen auf. Einige Beispiele sind Strömungen, die bei Extrusion, Beschichtung, Kontraktion und Fadenspinnen auftreten. Auch bei der Anwendung Kosmetischer Produkte fallen Dehneigenschaften ins Gewicht, die die sensorischen Eigenschaften beeinflussen oder auch je nach Anwendungen bestimmen können. Materialien die sich in einer Scherströmung ähnlich oder gleich verhalten, können sich in einer Dehnströmung völlig unterschiedlich verhalten. Die Kontraktion und das Abreißen eines Flüssigkeitsfadens, die mit dem CaBER™ analysiert werden, geben wertvolle Informationen über die physikalischen Eigenschaften eines Materials, die mit einem Rotationsrheometer nicht zugänglich sind. **Das "Capillary Breakup Extensional Rheometer" (CaBER™),** hergestellt von Thermo Haake, wurde durch die Firma Cambridge Polymer Group (CPG) entwickelt, basierend auf den Pionierarbeiten der russischen Wissenschaftler Entov, Rozhkov und Mitarbeiter auf dem Gebiet der "Fadenabriss Rheometrie". Das CaBER™ Dehnrheometer ist für die Untersuchung von Polymerlösungen, Suspensionen, Schmelzen, Klebstoffen, Emulsionen und anderen Materialien geeignet. Es ist sowohl als analytisches Messgerät für die Forschung als auch als Werkzeug für die Qualitätskontrolle einzusetzen. Das Messprinzip besteht in folgendem:
Eine geringe Probenmenge (< 0,2 ml) wird zwischen zwei runden Stempeln angebracht. Der obere Stempel wird extrem schnell hoch bewegt, hierbei wird die Probe augenblicklich gedehnt wobei ein instabiler Flüssigkeitsfaden entsteht. Nach Ende des Dehnvorganges entsteht in der Mitte des Fadens eine Dehnströmung, wobei die Dehnrate durch die Substanzeigenschaften bestimmt wird. Die Abnahme des Flüssigkeitsfadendurchmessers als Funktion der Zeit wird mit Hilfe eines Lasermikrometers gemessen. Die konkurrierenden physikalischen Effekte Oberflächenspannung, Elastizität, Viskosität und Massetransfer, die die Strömung bestimmen, können mittels Modellanalyse automatisch quantifiziert werden. Zusätzlich berechnet die Software noch Relaxationszeiten und die Fadenabbruchszeit.

Haargele können wie folgt gemessen werden:
Die Probe befindet sich zwischen zwei planparallelen Platten mit einem Durchmesser von 6mm. Der anfängliche Messspalt beträgt 2mm und wird während der Messung innerhalb von 500 ms linear auf 11 mm erhöht. Somit wird die Probe mit einem resultierende Hencky Strain von 1,7 gedehnt.

Für eine Beschreibung der erfindungsgemäßen Gele reicht die Darstellung Durchmesser versus Zeit vollständig aus. Es ist ein signifikanter Unterschied bei der zeitlichen Betrachtung des Durchmessers zu erkennen. Die erfindungsgemäße Probe zeigt einen bestehenden Faden, der mehrere Sekunden stabil und reproduzierbar messbar ist. Die herkömmliche Probe zeigt bereits nach 0,4 Sekunden keinen messbaren Durchmesser der Produktes bzw. Faden.

Nachfolgend sind einige Messungen mit ihren d(t)-Diagrammen wiedergegeben:

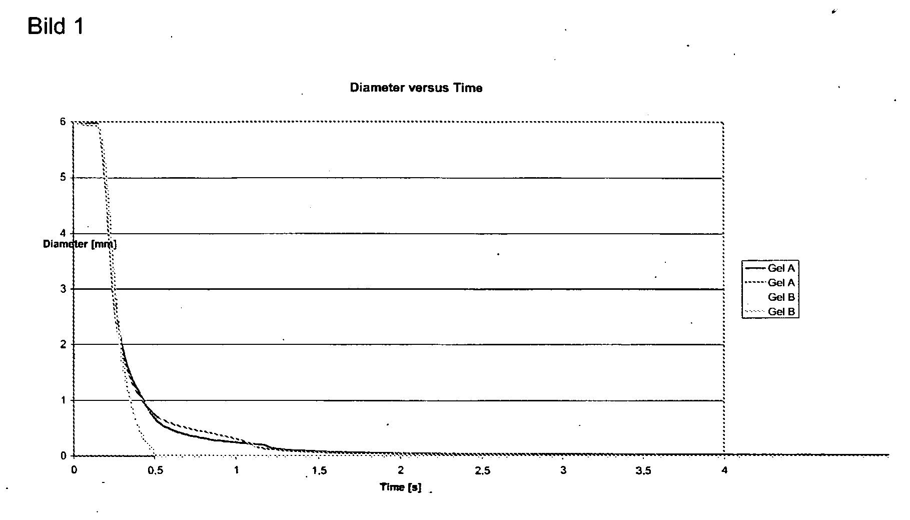

Gel A entspricht Beispiel 1; 2 Messungen.

Gel B entspricht Nivea Haar Styling Gel ultra stark (Herstelldatum KW 29 / 2005); 2 Messungen. Aus dem d(t)-Diagramm kann man erkennen, dass Gel B eine kurze Fadenabrisszeit von etwa 0, 5 s aufweist, während Gel A Fadenabrisszeiten von 2,5 bis 3,5 s aufweist.

### Vorteilhafte Ausführungsformen

Erfindungsgemäße Zubereitungen können ferner zusätzlich UV-Filter enthalten. Geeignete UV-Filter sind Benzophenone-4, Benzophenone-3 und Ethylhexylmethoxycinnamate (wie z.B. Escalol-Typen von ISP), Isoamyl-p-Methoxycinnamate (wie z.B. Neo Heliopan von Symrise).

Erfindungsgemäße Zubereitungen können ferner zusätzlich Silikonöle enthalten. Geeignete Silikonöle sind PEG-12 Dimethicone (wie z.B. DC 193 von Dow Corning), PEG-14 Dimethicone (wie z.B. ABIL B 8843 von Goldschmidt-Degussa), PEG/PPG-20-6 Dimethicone (wie z.B. ABIL B 88183 von Goldschmidt-Degussa), PEG/PPG-17/18 Dimethicone (wie z.B. DC Q-5220 Resin Modifier von Dow Corning).

Erfindungsgemäße Zubereitungen können ferner zusätzlich Neutralisationsmittel enthalten. Geeignete Neutralisationsmittel sind Hydroxide, deren Kation ein Ammonium oder ein Alkalimetall ist wie z. B. NaOH oder KOH sowie primäre, sekundäre oder tertiäre Amine, Aminoalkohole oder Ammoniak. Bevorzugt werden hier 2-Amino-2-methyl-1-propanol (AMP von Dow Chemical), Triethanolamine (TEA von BASF) oder N,N,N',N'-Tetrakis (2-hydroxypropyl) ethylenediamine (Neutrol TE von BASF) eingesetzt.

Erfindungsgemäße Zubereitungen können ferner zusätzlich (bitte alle Stoffgruppen angeben) enthalten.

Geeignete nichtionische Polymere sind z.B. Homopolymere des Vinylpyrrolidons, z.B. Luviskol K von BASF oder Homopolymere des N-Vinylformamids z.B. PVF von National Starch.

Weitere geeignete Polymere sind Copolymerisate aus Vinylpyrrolidon und Vinylacetat z.B. Luviskol VA Typen von BASF, Copolymere aus Vinylpyrrolidon und Dimethylaminoethylmethacrylat, Terpolymere aus Vinylcaprolactam, Vinylpyrrolidon und Dimethylaminomethacrylat sowie Copolymere aus Vinylpyrrolidon, Methacrylamide und Vinyl Imidazol (z.B. Luviset Clear von BASF)

Geeignete kationische Polymere mit basischen Gruppen sind z.B. Copolymere von aminsubstituierten Vinylmonomeren und nicht aminsubstituierten, nicht kationischen Monomeren. Aminsubstituierte Vinylmonomere sind z.B.Dialkylaminoalkylacrylat, Dialkylaminoalkylmethacrylat, Monoalkylaminoalkylacrylat, Monoalkylaminoalkyl-methacrylat, wobei die Alkylgruppen dieser Monomere vorzugsweise niedere Alkylgruppen wie z.B. C1-C7- Alkylgruppen, besonders bevorzugt C1- C3-Alkylgruppen sind.

Geeignete Polymere sind unter den Bezeichnungen Polyquaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/- Dimethylaminoethylmethacrylat Copolymer (Polyquaternium-11) z.B. Gafquat 755 von ISP oder Luviquat PQ-11 von BASF, Homo- und Copolymere von Dimethyldiallylammoniumchlorid (Polyquaternium-6, -7 oder-22), quaternisierte Hydroxy-ethylcellulose (Polyquaternium-10) oder quaternisierte Guarderivate.

Nicht aminsubstituierte, nichtkationische Comonomere sind z.B. Acrylamid, Methacrylamid, Alkyl- und Dialkylacrylamid, Alkyl- und Dialkylmethacrylamid, Acrylacrylat, Alkylmethacrylat, Vinylcaprolacton, Vinylpyrrolidon, Vinylester, Vinylalkohol, Maleinsäreanhydrid, Propylenglycol oder Ethylenglycol, wobei die Alkylgruppen dieser Monomere vorzugsweise niedere Alkylgruppen wie z.B. C1-C7- Alkylgruppen, besonders bevorzugt C1-C3-Alkylgruppen sind.

Weitere kationische Polymere sind: Copolymere von Hydroxyethylcellulose und Diallyldimethyl Ammonium Chloride (INCI Polyquaternium-4) z.B. Celquat L 200 von National Starch

Beispiele für synthetische Verdicker sind: Polymere und Copolymere der Acrylsäure, Polymere und Copolymere der Methacrylsäure, Polymere und Copolymere der Crotonsäure oder Polymere und Copolymere der Salze oder Ester der Acrylsäure, Methycrylsäure oder Crotonsäure.

Homopolymere der Acrylsäure mit einem Molekulargewicht von 2000000 bis 6000000 wie z.B. Handelsprodukt Carbopole. Weiter Verdicker werden vertrieben unter den Namen Carbopol 980, Carbopol Ultrez 10

Weitere Beispiele für geeignete Copolymere sind beispielsweise das Acryl- oder Methacrylsäure/Acryl oder Methacrylsäurepolyethoxyalkylester Copolymer (INCI: Acrylates/ Beheneth-25 Methacrylate Copolymer) wie von der Fa. Rohm und Haas unter der Bezeichnung Aculyn-28 vertrieben wird. Geeignet sind auch Acryl- oder Methacrylsäure/Itaconsäure-polyethoxyalkyester Copolymer (INCI: Acrylates/Steareth-20 Itaconate Copolymer und Acrylates/Ceteth-20 Itaconate Copolymer) wie von Fa. National Starch unter der Bezeichnung Structure 2001 und Structure 3001 vertrieben.

Ferner können als synthetische Verdicker Polyethylenglycole oder Polypropylenglycole verwendet werden. Besonders bevorzugt sind hierbei hochmolekulare Polyethylenglycole mit einher Molmasse von 100000 bis 7000000 g/mol.

Weitere natürliche oder natürlich modifizierte Verdickertypen sind Polysaccharide und deren Derivate oder deren Hydrolysate wie Cellulose oder Cellulosederivate, wie ethoxylierte Cellulosen, insbesondere Cellulose Gums oder Hydroxyalkylcellulosen.

Geeignete sind auch weitere Verdicker auf natürlicher Basis sind die Gums wie Xanthan Gum oder Guar Gum.

Geeignete anionische Polymere sind Homo- oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbareVerbindungen, welche mindestens eine Säuregruppe besitzen., insbesondere Carboxyvinylmonomere. Geeignete Säuregruppen enthaltende Monomere sind beispielsweise Acrylsäure, Meth-acrylsäure, Crotonsäure, Maleinsäure bzw. Maleinsäureanhydrid oder deren Monoester, Aldehydcarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethylacrylamid, Alkylacrylat, Alkylmethacrylat, Vinylcaprolactam, Vinylpyrrolidon, Vinylester, Vinylalkohol, Propylenglycol oder Ethylenglycol, aminsubstituierte Vinylmonomere wie z.B. Dialkylaminoalkylacrylat, Dialkylaminoalkylmethacrylat , Monoalkyl-aminoalkylacrylat und Monoalkylaminoalkylmethacrylat.

Weitere Polymere mit Säuregruppen sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidonen, Homo-polymere der Crotonsäure Acrylamiden, Methacrylamiden und Vinylpyrrolidonen, Homo-polymere der Crotonsäure sowie Copolymere der Crotonsäure mit Monomeren aus-gewählt aus Vinylestern, Acrylsäure- oder Methacrylsäureestern, Acrylamiden und Methacrylamiden.

Geeignete anionische Polymere sind auch in Wasser lösliche oder dispergierbare anionische Polyurethane z.B. Luviset PUR von BASF oder Gemische derselben z.B. DynamX von National Starch.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Nicht erfindungsgemäße Beispiele sind mit einem Stern im Tabellenkopf gekennzeichnet.

### Beispiele

Folgende Handelsprodukte wurden eingesetzt:
1) Luviskol VA-Typen von BASF oder PVPNA-Typen von ISP
2) Luviskol K-Typen von BASF
3) Luviquat FC 370 von BASF
4) Gafquat 755N von ISP
5) Celquat L200 von National Starch
6) Luviquat Hold von BASF
7) Polyox-Typen von Dow Chemical z.B. Polyox WSR-301
8) Carbopol 980 oder Carbopol Ultrez 10 von Noveon
9) Klucel EF oder HF von Hercules
10) Natrosol 250 von Hercules
11) Jaguar-Typen von Rhodia
12) Dehyquart A-CA von Cognis oder CTAC 6025 von KCI
13) Carbopol Ultrez 21 oder Carbopol ETD 2020 oder Carbopol 1382 von Noveon
14) Luviset Clear von BASF
15) Aculyn 28 von Rohm&Haas
16) Structure 3001 von National Starch
17) Keltrol F von Kelco
18) DynamX von National Starch
19) Aquaflex-30 von ISP
20) Synthalen W 2000 von 3V Sigma
21) Carbopol Aqua SF-1 von Noveon
22) Methocel E4M von Dow Chemical

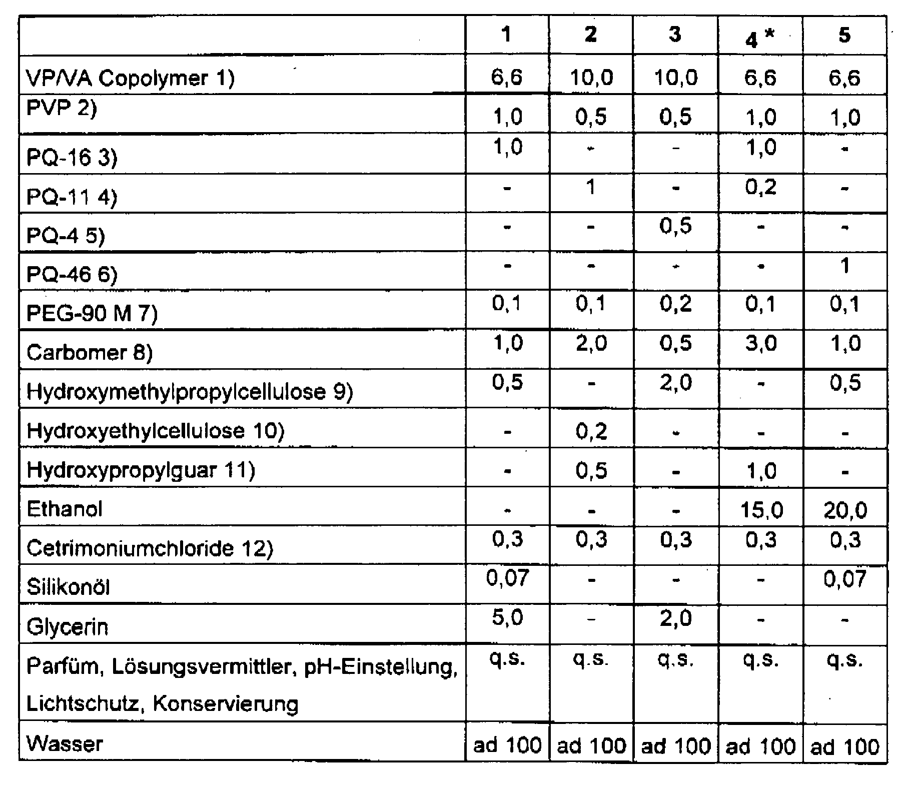

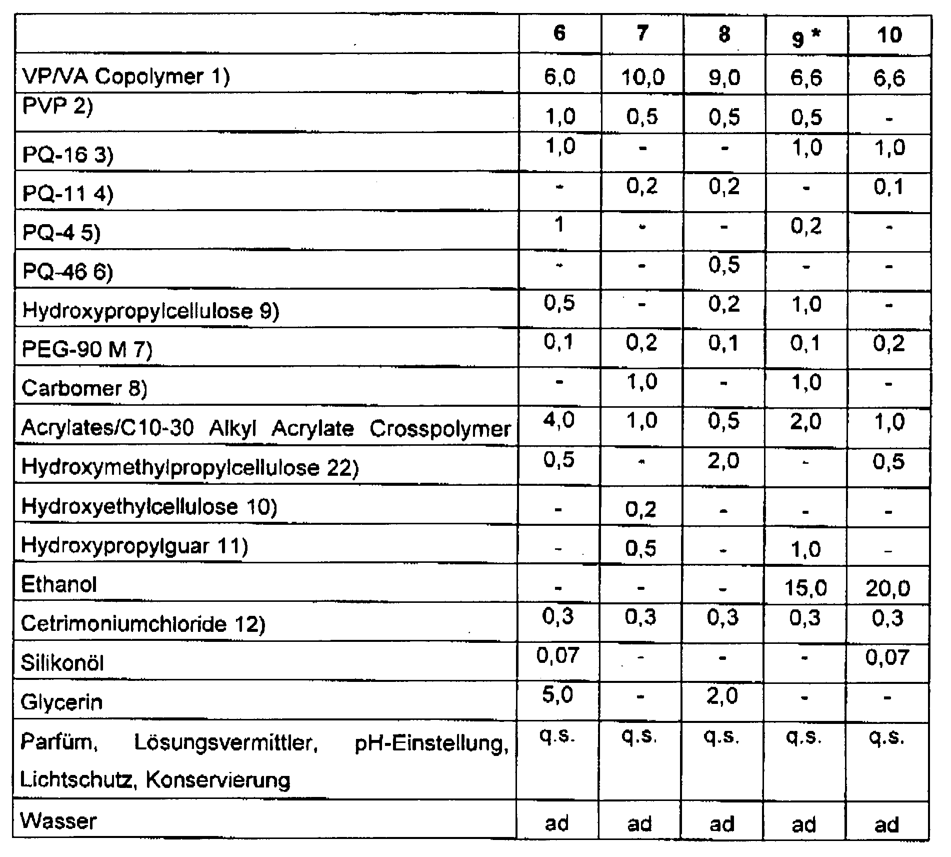

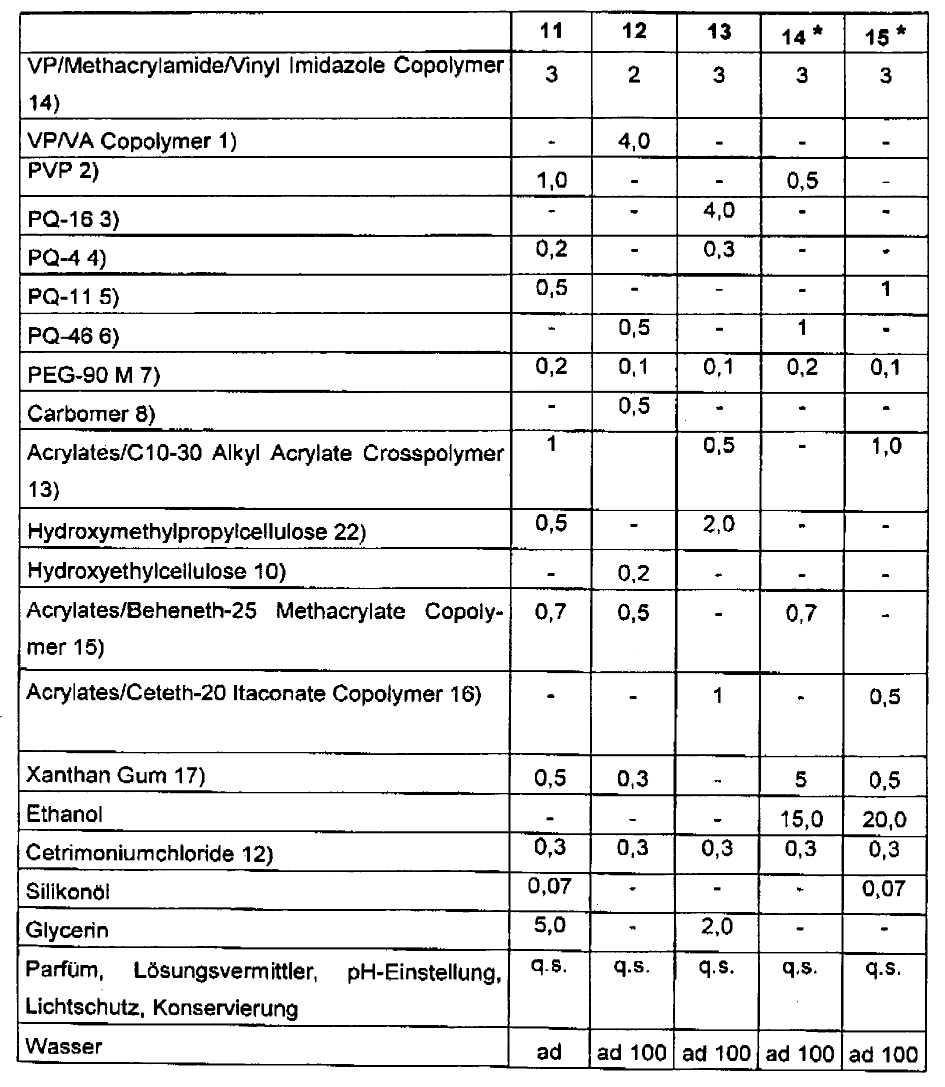

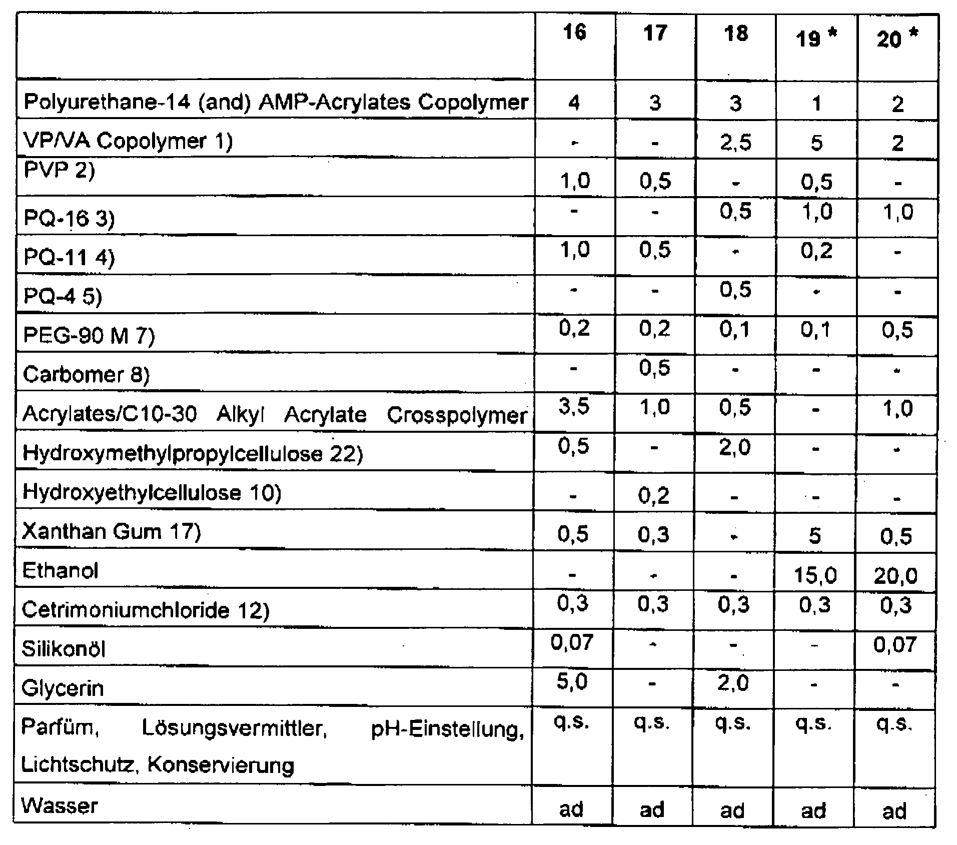

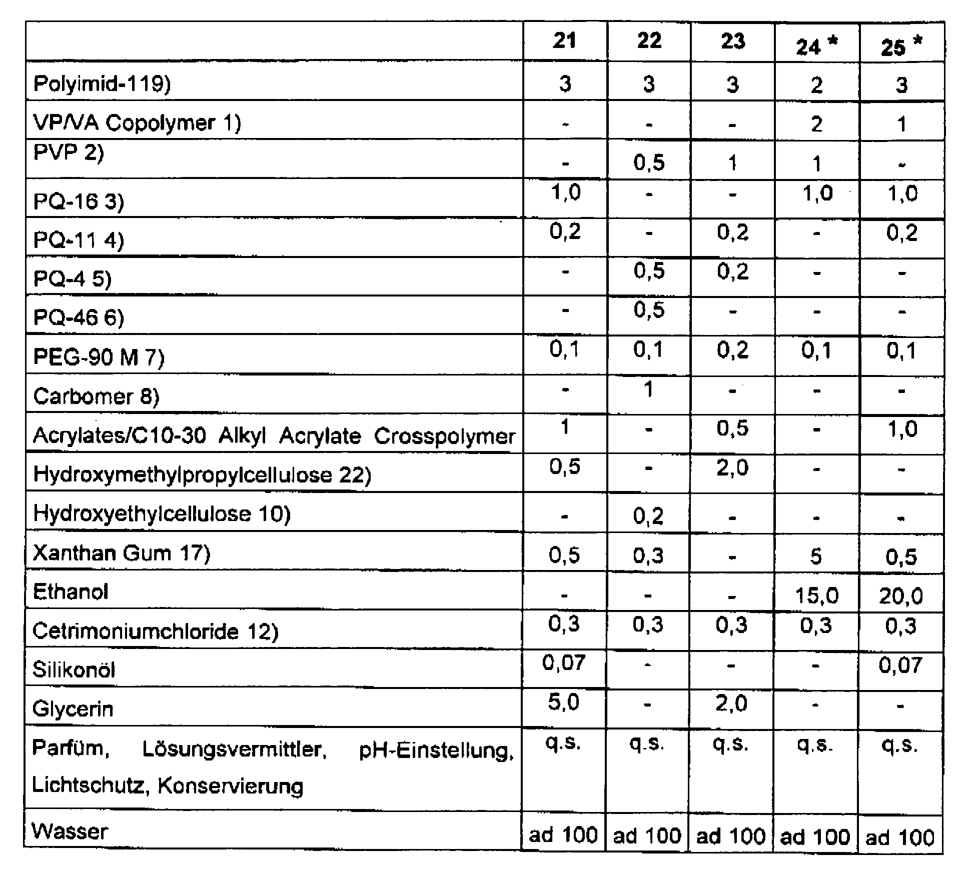

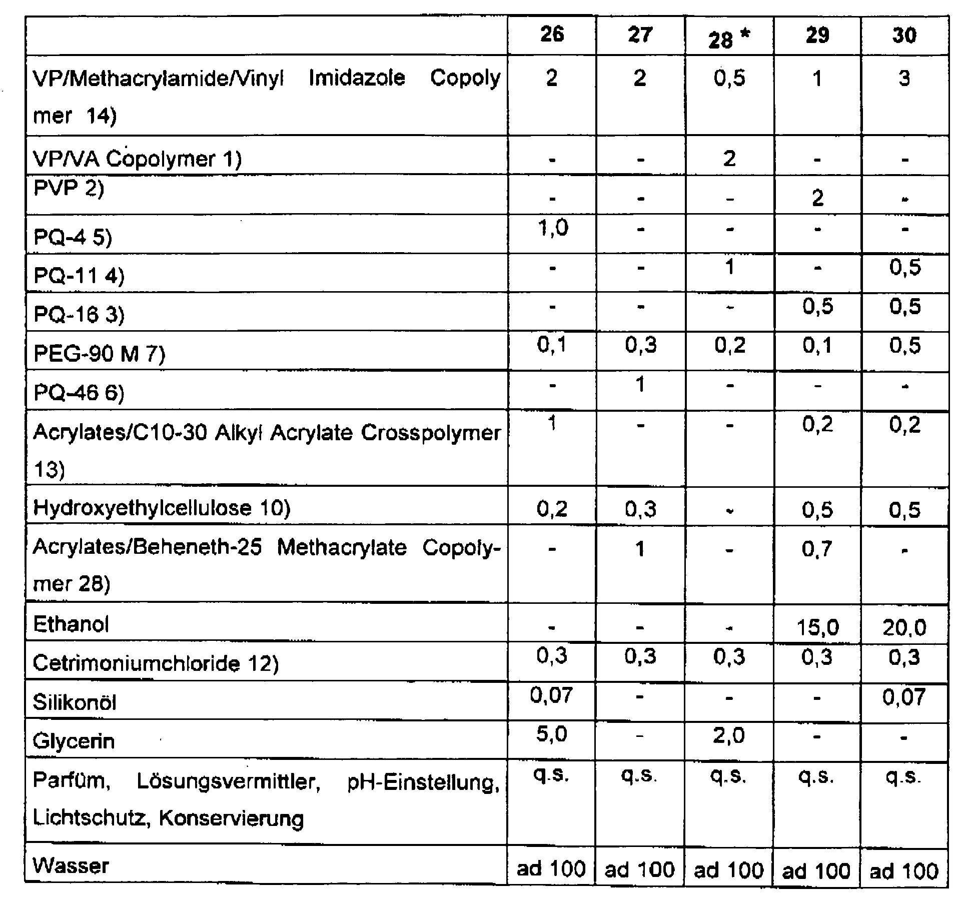

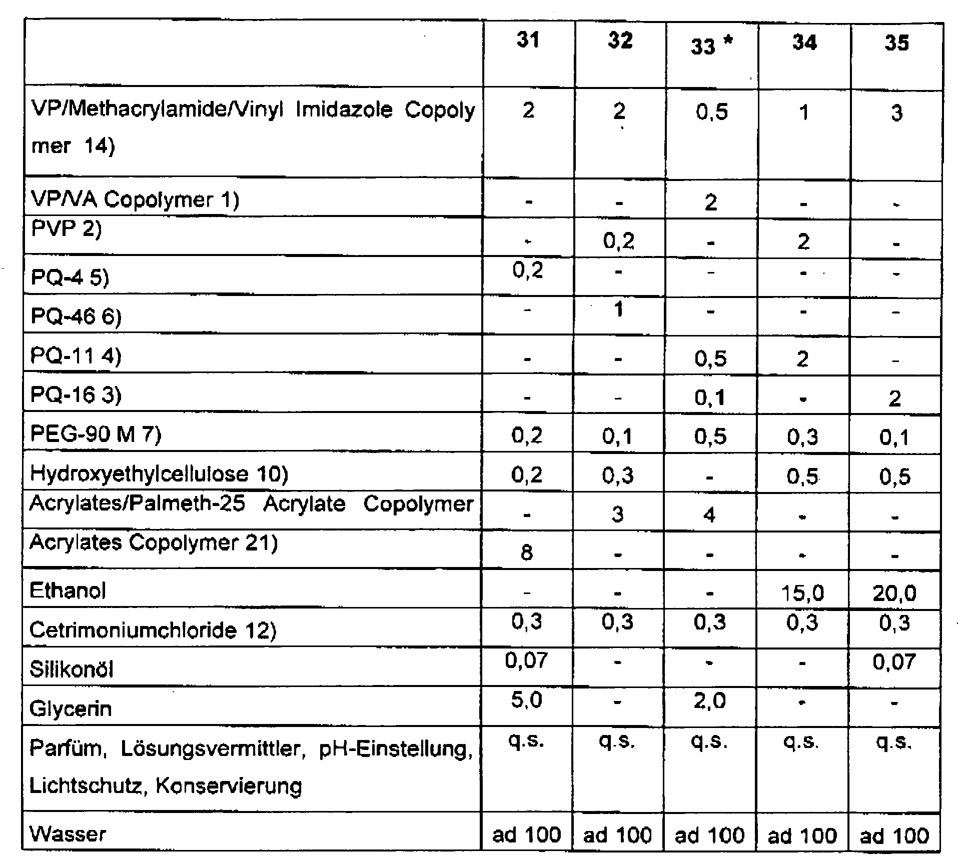

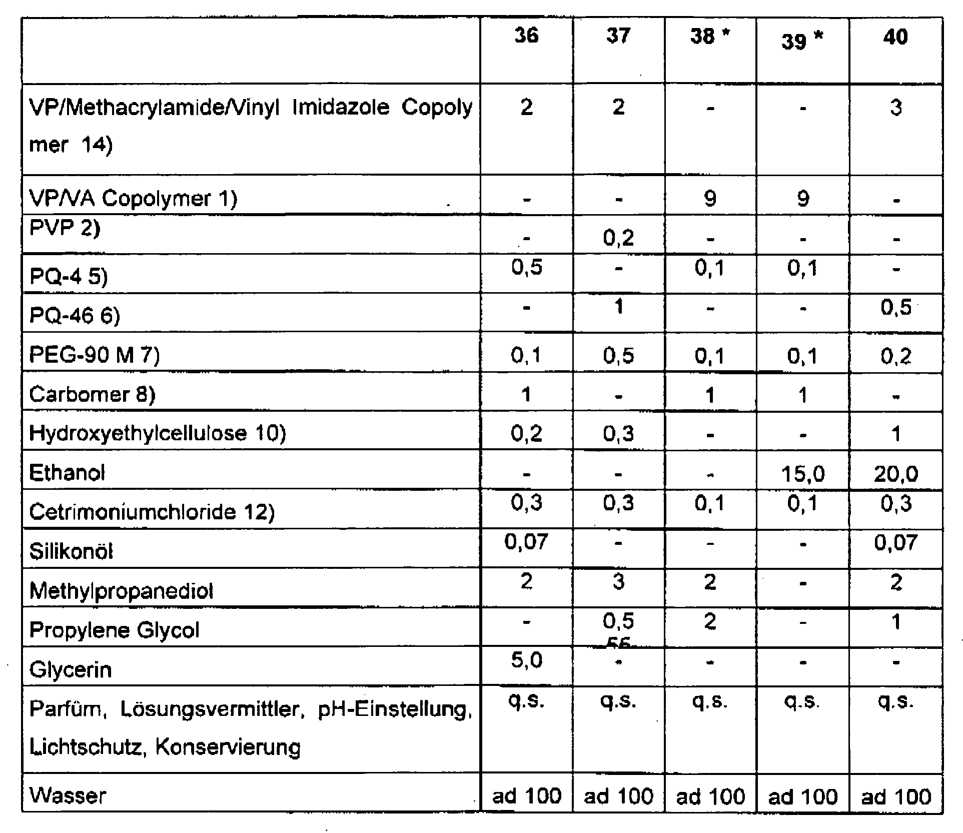

## Patentansprüche

1. Gelförmige kosmetische Zubereitung zur Frisurgestaltung enthaltend
- ein anionisches verdickendes Polymer,
- ein oder mehrere filmbildende Polymere, bevorzugt ein VP/VA-Copolymer,
- als konditioniemdes Polymer eine kationische Zellulose,
- ein hochmolekulares Polyethylenglycol mit einer Molmasse von 3000000 bis 5000000 g/mol, bevorzugt 3500000 bis 4500000 g/mol,
- Wasser, **dadurch gekennzeichnet, dass** die Fadenabrisszeit der Zubereitung gemessen mit einem Capillary Breakup Extensional Rheometer (CaBER™, Thermo Haake) zwischen 1 s und 5,0 s, bevorzugt zwischen 1,5 s und 4,0 , besonders bevorzugt zwischen 2,0 und 3,5 liegt.

2. Zubereitung nach Patentanspruch 1 **dadurch gekennzeichnet, dass** zusätzlich Alkohol enthalten ist.

3. Zubereitung nach einem der vorangehenden Patentansprüche **dadurch gekennzeichnet, dass** der Gehalt an verdickendem Polymere 0,1 bis 5 Gew.% bezogen auf den Aktivgehalt an Polymer beträgt.

4. Zubereitung nach einem der vorangehenden Patentansprüche **dadurch gekennzeichnet, dass** der Gehalt an filmbildendem Polymer 0,1 bis 5 Gew.% bezogen auf den Aktivgehalt an Polymer beträgt.

5. Zubereitung nach einem der vorangehenden Patentansprüche **dadurch gekennzeichnet, dass** der Gehalt an konditionierndem Polymer 0,1 bis 3 Gew.% bezogen auf den Aktivgehalt an Polymer beträgt.

6. Zubereitung nach einem der vorangehenden Patentansprüche **dadurch gekennzeichnet, dass** der Gehalt an hochmolekularem Polyethylenglycol 0,1 bis 2 Gew.% beträgt.

7. Zubereitung nach einem der vorangehenden Patentansprüche **dadurch gekennzeichnet, dass** der Gehalt an Öl abzüglich Parfümölen 0 bis 0,2 Gew.% beträgt.

8. Zubereitung nach einem der vorangehenden Patentansprüche **dadurch gekennzeichnet, dass** der Gehalt an Tensiden 0,1 bis 2 Gew.% beträgt, falls in der Formulierung Parfümöl enthalten ist, andernfalls **dadurch gekennzeichnet, dass** der Gehalt an Tensiden 0 bis 0,5 Gew.% beträgt.

9. Zubereitung nach einem der vorangehenden Patentansprüche **dadurch gekennzeichnet, dass** sie Carbomer, Polyquatemium-4, VPNA Copolymer und PEG-90 M enthält.

10. Zubereitung nach einem der vorangehenden Patentansprüche **dadurch gekennzeichnet, dass** die Viskosität im Bereich 100 bis 300 dPas, bevorzugt 150 bis 250 dPas (gemessen mit Haake VT-02 Viskotester) liegt.

## Claims

1. Gel-like cosmetic preparation for hairstyling comprising
- an anionic thickening polymer,
- one or more film-forming polymers, preferably a VPNA copolymer,
- as conditioning polymer, a cationic cellulose,
- a high molecular weight polyethylene glycol with a molar mass of 3 000 000 to 5 000 000 g/mol, preferably 3 500 000 to 4 500 000 g/mol,
- water, **characterized in that** the filament break-up time of the preparation measured using a capillary breakup extensional Rheometer (CaBER™, Thermo Haake) is between 1 s and 5.0 s, preferably between 1.5 s and 4.0, particularly preferably between 2.0 and 3.5.

2. Preparation according to Patent Claim 1, **characterized in that** alcohol is additionally present.

3. Preparation according to one of the preceding patent claims, **characterized in that** the content of thickening polymer is 0.1 to 5% by weight, based on the active content of polymer.

4. Preparation according to one of the preceding patent claims, **characterized in that** the content of film-forming polymer is 0.1 to 5% by weight, based on the active content of polymer.

5. Preparation according to one of the preceding patent claims, **characterized in that** the content of conditioning polymer is 0.1 to 3% by weight, based on the active content of polymer.

6. Preparation according to one of the preceding patent claims, **characterized in that** the content of high molecular weight polyethylene glycol is 0.1 to 2% by weight.

7. Preparation according to one of the preceding patent claims, **characterized in that** the content of oil minus perfume oils is 0 to 0.2% by weight.

8. Preparation according to one of the preceding patent claims, **characterized in that** the content of surfactant is 0.1 to 2% by weight if perfume oil is present in the formulation, otherwise **characterized in that** the content of surfactant is 0 to 0.5% by weight.

9. Preparation according to one of the preceding patent claims, **characterized in that** it comprises carbomer, polyquaternium-4, VPNA copolymer and PEG-90 M.

10. Preparation according to one of the preceding patent claims, **characterized in that** the viscosity is in the range 100 to 300 dPas, preferably 150 to 250 dPas (measured using a Haake VT-02 Viskotester).

## Revendications

1. Composition cosmétique sous forme de gel pour le coiffage, contenant
- un polymère épaississant anionique,
- un ou plusieurs polymères filmogènes, de préférence un copolymère de VPNA,
- comme polymère de conditionnement une cellulose cationique,
- un polyéthyléneglycol de haut poids moléculaire, présentant une masse molaire de 3 000 000 à 5 000 000 g/mole, de préférence de 3 500 000 à 4 500 000 g/mole,
- de l'eau,
**caractérisée en ce que** le temps de rupture du fil de la composition, mesuré avec un rhéomètre extensionnel de rupture capillaire (Capillary Breakup Extensional Rheometer- CaBER^{™}, Thermo Haake), est situé entre 1 s et 5,0 s, de préférence entre 1,5 s et 4,0, de manière particulièrement préférée entre 2,0 et 3,5.

2. Composition selon la revendication 1, **caractérisée en ce qu'**en outre, un alcool est contenu.

3. Composition selon l'une quelconque des revendications précédentes, **caractérisée en ce que** la teneur en polymère épaississant est de 0,1 à 5% en poids par rapport à la teneur active en polymère.

4. Composition selon l'une quelconque des revendications précédentes, **caractérisée en ce que** la teneur en polymère filmogène est de 0,1 à 5% en poids par rapport à la teneur active en polymère.

5. Composition selon l'une quelconque des revendications précédentes, **caractérisée en ce que** la teneur en polymère de conditionnement est de 0,1 à 3% en poids par rapport à la teneur active en polymère.

6. Composition selon l'une quelconque des revendications précédentes, **caractérisée en ce que** la teneur en polyéthylèneglycol de haut poids moléculaire est de 0,1 à 2% en poids.

7. Composition selon l'une quelconque des revendications précédentes, **caractérisée en ce que** la teneur en huile, déduction faite des huiles parfumées, est de 0 à 0,2% en poids.

8. Composition selon l'une quelconque des revendications précédentes, **caractérisée en ce que** la teneur en agents tensioactifs est de 0,1 à 2% en poids, lorsque de l'huile parfumée est contenue dans la formulation, sinon, **caractérisée en ce que** la teneur en agents tensioactifs est de 0 à 0,5% en poids.

9. Composition selon l'une quelconque des revendications précédentes, **caractérisée en ce qu'**elle contient un carbomère, du Polyquaternium-4, un copolymère de VPNA et du PEG-90 M.

10. Composition selon l'une quelconque des revendications précédentes, **caractérisée en ce que** la viscosité est située dans la plage de 100 à 300 Pa.s, de préférence de 150 à 250 Pa.s (mesurée avec un testeur de viscosité Haake VT-02).
